# EUROPEAN PATENT SPECIFICATION

(11) **EP 3 372 251 B1**
(45) Date of publication and mention of the grant of the patent: **02.12.2020**
(21) Application number: 18160986.8
(22) Date of filing: 09.03.2018
(51) Int. Cl.: A61L 2/04, A61L 2/16, A61L 2/26, B08B 3/02

(54) **MACHINE FOR TREATING OBJECTS, IN PARTICULAR WASHING, THERMAL DISINFECTION AND/OR STERILIZATION OF OBJECTS**
MASCHINE ZUR BEHANDLUNG VON OBJEKTEN, INSBESONDERE ZUM WASCHEN, ZUR THERMISCHEN DESINFEKTION UND/ODER ZUM STERILISIEREN VON OBJEKTEN
MACHINE DE TRAITEMENT D'OBJETS, EN PARTICULIER LE LAVAGE, LA DÉSINFECTION THERMIQUE ET/OU LA STÉRILISATION D'OBJETS

(30) Priority: 10.03.2017 IT 201700027052
(43) Date of publication of application: 12.09.2018
(73) Proprietor: Steelco S.p.A., 31039 Riese Pio X (IT)
(72) Inventor: Capovilla, Ivone, 31037 Loria (TV) (IT)
(74) Representative: Petraz, Gilberto Luigi

(56) References cited:
- WO-A1-2015/145383
- US-A- 5 641 260

## Description

### FIELD OF THE INVENTION

The present invention concerns a machine for treating objects, in particular a so-called "tunnel" treatment machine, in which successive steps of the treatment of objects can be carried out in one or more treatment chambers in which relative treatment zones are disposed one after the other.

By the term treatment it is possible to understand "in their entirety", both pretreatment operations of the objects, such as pre-washing, with hot or cold water and/or with detergents or other chemical products, and also washing operations proper, and also drying operations. Also included in the same term "treatment" are thermal disinfection, sterilization, generally in an autoclave, and decontamination by means of particularly aggressive and dangerous decontaminating substances, detergents and/or chemical agents, such as for example peracetic acid.

By way of example, the objects that can be treated in the treatment machine in question can be instruments used in the health sector, in the laboratory, for analysis or for research, instruments used in the pharmaceutical sector, or instruments in the medical sector, surgical instruments or comparable or similar instruments, without excluding, however, the application of the present invention to the treatment of objects in general.

### BACKGROUND OF THE INVENTION

It is known to make machines for treating objects, generally contained in one or more object-holder containers, such as baskets or suchlike, which are fed inside at least one suitable treatment chamber, by feed means, for example a belt, where the desired treatment cycle is performed.

Normally, at least one door is provided, associated with an entrance aperture, which is mobile and driven by an opening and closing mechanism to assume a high or open position in which it allows the entrance of the objects to be treated in the treatment chamber, and a low or closed position in which, in cooperation with fluidic sealing means, it seals the washing chamber from the outside.

In "single-door" treatment machines, the entrance and exit of the containers and of the treated objects contained therein take place through the same door. In "pass-through" treatment machines, two doors are provided, that is, one door associated with an entrance aperture and one door associated with an exit aperture, normally in a position opposite the entrance aperture, respectively for the entrance and exit of the containers and of the treated items contained therein.

Inside the machine's treatment chamber, several object-holder containers can also be inserted, located in sequence one after the other in the chamber.

In the case of a "single-door" treatment machine, a rollerway is provided on which the object-holder containers are rested and situated in correspondence with the door of the machine. From the door, therefore, the containers enter with the objects to be treated and the containers with the treated objects come out.

In the case of a "pass-through" treatment machine, generally two rollerways are provided, positioned on diametrically opposite sides of the machine, of which one side has an entrance door of the containers with objects to be treated and, the opposite side, has an exit door of the containers with the treated objects.

In particular, the treatment machines configured for sterilization are generally machines with a deep treatment chamber, for example even a few meters long, therefore, in such machines, multiple object-holder containers disposed in sequence are loaded. In the sterilization treatment relatively high temperatures are reached, for example up to about 140°C, therefore, it is good practice if the sterilization machine does not have, inside the treatment chamber, automatic movement means for the object-holder containers and/or sensors to detect their position.

To load the treatment chamber and start the intended treatment cycle, the object-holder containers are thrust and translated one after the other through the loading door of the treatment chamber.

In the case, for example, of two containers located in sequence, a first container will be inserted into the chamber, then, once the insertion is completed, a second container will be positioned after the first container, which, following the thrust of the second container, will occupy a bottom zone of the chamber.

Once the containers have been inserted in sequence, the chamber is closed and the treatment cycle begins. In the event that, for any reason whatsoever, for example a fault, an alarm or other, the intended cycle is not completed and it becomes necessary to remove the two containers located in the chamber, the loading door is opened so that they can be extracted.

It should be noted that in the case of premature interruption of a treatment cycle, for example due to a fault or alarm, even in a "pass-through" machine it is necessary to unload the containers from the loading door, as the unloading door automatically blocks. This blocking of the unloading door is mainly due to the fact that the zone of the treated objects, located downstream of the machine, must not in any way enter into communication with the zone of the objects still to be treated.

To proceed with unloading the containers therefore, the second container, inserted last and positioned in proximity to the loading door, can be extracted without difficulty, but the first container inserted, which is at the bottom of the chamber, is more difficult to extract.

Normally, in order to extract a container that is in proximity to the bottom of a treatment chamber, an operator has to enter into the chamber until he can grasp the object-holder container and pull it toward him in order to remove it. This operation, as can be imagined, proves rather difficult, since the environment of the treatment chamber, as we said above, can be extremely hot and furthermore the container can have a certain weight, since it is loaded with the objects to be treated.

In a treatment machine provided with a treatment chamber in which several object-holder containers disposed in sequence are positioned, therefore, it can be extremely difficult and not very safe for operators to remove the object-holder containers, in the event of premature interruption of the treatment cycle.

Document WO-A-2015/145383 describes a device to introduce and extract containers with respect to a sterilization machine along a direction of feed, comprising a container loading plane defining a movement path, and motorized linear guide members disposed along the container loading plane and configured to move the containers in a guided manner along the movement path in the direction of feed. The device also comprises an auxiliary guide extension connected to the container loading plane and mobile between a first retracted position, in which it is comprised in the bulk of the container loading plane, and a second operating position, in which it is at least partly protruding from the container loading plane to define an extension of the movement path.

Although the device has undoubted advantages in moving the baskets or containers, in the sense that it prevents possible blockages thereof during moving, it has some limitations with regard to the possibility of automatic attachment of a plurality of containers disposed in sequence, so that the sterilizing machine provided with this device has problems similar to those mentioned above.

Other limitations and disadvantages of conventional solutions and technologies will be clear to a person of skill after reading the remaining part of the present description with reference to the drawings and the description of the embodiments that follow, although it is clear that the description of the state of the art connected to the present description must not be considered an admission that what is described here is already known from the state of the prior art.

There is therefore a need to obtain a machine for treating objects, in particular for washing, thermal disinfection and/or sterilization of objects, which can overcome at least one of the disadvantages of the prior art.

In particular, one purpose of the present invention is therefore to provide a machine for treating objects which allows the automatic attachment of several object-holder containers disposed in sequence upon their insertion into the treatment chamber, so that, acting on the slider nearest to the loading door, in the opposite direction to the insertion direction, all the object-holder containers present in the chamber can be extracted, and therefore also the object-holder container located at the bottom of the treatment chamber and in the farthest position from the loading door, thanks to the fact that it is attached to the object-holder container closest to the loading door.

Another purpose of the present invention is to provide a machine for treating objects which has a device of a mainly mechanical type to automatically attach the sliders upon their insertion.

Another purpose of the present invention is to provide an effective and automatic reciprocal attachment apparatus for several object-holder containers, which allows to translate the object-holder containers simultaneously at least in a step where they are extracted from the treatment chamber and at least as far as the loading door of any machine for treating objects.

Another purpose of the present invention is to perfect a quick, automatic and safe method for the reciprocal attachment of several object-holder containers.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purpose, a machine for treating objects, in particular washing, thermal disinfection and/or sterilization of objects according to the invention comprises at least a treatment chamber configured to house a plurality of object-holder containers located in sequence and comprising opposite lateral walls.

According to possible embodiments, the machine comprises at least a releasable connection device of the object-holder containers, associated with at least one of the object-holder containers and configured to cooperate with activation means positioned on the lateral walls inside the treatment chamber, so that, inserting an object-holder container in the treatment chamber after at least a first object-holder container already inserted in the chamber and moving the object-holder containers toward the inside of the chamber, the releasable connection device is automatically activated by the activation means in order to reciprocally connect the object-holder containers, so as to allow a simultaneous extraction, acting on the last object-holder container inserted.

In possible embodiments, the device comprises at least a sliding block associated with at least a first head and/or tail bar of a object-holder container and provided with an attachment element able to engage the first bar and at least a second bar of another object-holder container, following the translation of the sliding block with respect to the first bar, the sliding block is made to translate in one direction or the other with respect to the bar by means of the activation means.

According to another aspect of the invention, the machine comprises, on the lateral walls of the chamber, de-activation means of the releasable connection device, so as to automatically separate the object-holder containers during their extraction step from the treatment chamber.

Preferably the de-activation means are positioned in proximity to an entrance aperture of the treatment chamber.

In some embodiments, the releasable connection device is provided both at the tail and at the head of each object-holder container.

If a connection device is provided both at the head and at the tail of each object-holder container, the activation means and the de-activation means of the connection devices can be staggered at least in height in the lateral walls inside the treatment chamber.

In some embodiments, the sliding block can comprise at one end at least a first rolling element able to engage with the activation means and, at the other end, at least a second rolling element able to engage with the de-activation means.

The first and second rolling elements can be alternatively protruding from opposite sides of the object-holder container, in particular longitudinal sides.

The activation means can comprise at least a cam profile which can engage with the first rolling element of the releasable connection device.

The de-activation means can comprise at least another cam profile which can engage with the second rolling element of the releasable connection device.

The invention also concerns a connection device of a plurality of object-holder containers located in sequence in a treatment machine, in particular for washing, thermal disinfection and/or sterilization of objects.

According to possible embodiments, the connection device comprises at least a sliding block associated with at least a first head and/or tail bar of a first object-holder container and provided with an attachment element able to engage the first bar and at least a second bar of a second object-holder container, following a translation of the sliding block with respect to the first bar; the sliding block is made to translate in one direction or the other with respect to the bar by means of activation means positioned in the machine.

The invention also concerns an object-holder container usable in a machine for treating objects, in particular for washing, thermal disinfection and/or sterilization of objects, which comprises at least one connection device as defined above.

The invention also concerns a method to connect a plurality of object-holder containers located in sequence in a treatment machine, in particular for washing, thermal disinfection and/or sterilization of objects.

In possible embodiments, the method provides: to insert a first object-holder container in a treatment chamber; to insert at least a second object-holder container in the treatment chamber after the first object-holder container has already been inserted in the treatment chamber and to move the object-holder containers toward the inside of the treatment chamber; at least one of the object-holder containers is provided with a releasable connection device automatically activated by activation means disposed on the lateral walls inside the treatment chamber to reciprocally connect said object-holder containers, so as to allow a simultaneous extraction, acting on the last object-holder container inserted.

In possible embodiments of the method, the device can comprise at least a sliding block associated with at least a first head and/or tail bar of a object-holder container and provided with an attachment element able to engage the first bar and at least a second bar of another object-holder container following a translation of the sliding block with respect to the first bar; the sliding block is made to translate in one direction or the other with respect to the bar by means of activation means.

These and other aspects, characteristics and advantages of the present disclosure will be better understood with reference to the following description, drawings and attached claims. The drawings, which are integrated and form part of the present description, show some forms of embodiment of the present invention, and together with the description, are intended to describe the principles of the disclosure.

The various aspects and characteristics described in the present description can be applied individually where possible. These individual aspects, for example aspects and characteristics described in the specification or in the attached dependent claims, can be the object of divisional applications.

It is understood that any aspect or characteristic, that is discovered to be already known during the patenting process, shall not be claimed and shall be the object of a disclaimer.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a schematic three-dimensional view of a machine for treating objects according to the invention, in which an object-holding container is about to be introduced;
- fig. 2 is a schematic front view of a treatment chamber of the present machine, in which an object-holding container is about to be introduced;
- fig. 3 is a schematic plan view of a pair of object-holding containers located in sequence, of which a first object-holding container has already been inserted in the treatment chamber;
- figs. 4a-4e are schematic three-dimensional views of a connection and disconnection sequence of the two object-holding containers.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We shall now refer in detail to the various embodiments of the present invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

With reference to the attached drawings, a treatment machine 10 comprises at least one treatment chamber 11 in which a plurality of object-holder containers, disposed in sequence, for example, the object-holder containers 12 and 13 can be positioned.

The object-holder containers 12 and 13 may be in the form of baskets or suchlike.

Typically, the object-holder containers 12 and 13 comprise a frame 14 provided with at least one head bar 15, see for example the head bar 15 of the object-holder container 13, and at least one tail bar 16, see for example the tail bar 16 of the object-holder container 12.

The bars 15 or 16 are directed in a direction T substantially transverse with respect to a direction L for the insertion and extraction of the object-holder containers 12 and 13 into and from the chamber 11.

The frame 14 also comprises at least one pair of sides 20 and 21, substantially directed in the direction L. The sides 20 and 21 are preferably made at the same height.

The sides 20 and 21 are preferably disposed at the same height as the bars 15 and 16, therefore in proximity to the base of the object-holder containers 12 and 13.

The provision of two or more object-holder containers 12 and 13 disposed in sequence is useful, for example, in the event that the user has problems of bulk for the operations of inserting and extracting the object-holder containers from the treatment machine 10.

In fact, the length of the object-holder container or containers 12 and 13 located in sequence is normally such that the whole length of the treatment chamber 11 is substantially occupied.

Hereafter in the description we will refer, by way of non-restrictive example, to the example shown of two object-holder containers 12 and 13 located in sequence, and to a "single door" treatment machine. As can easily be seen, however, the present invention can also concern a "pass-through" treatment machine, since, in the event of failure, breakdown, error in the cycle or suchlike, as mentioned above, the unloading door is automatically blocked so that, in fact, the loading aperture would also be used to remove the object-holder containers.

The treatment chamber 11 will be provided inside it with means, not shown in the drawings, useful to carry out a specific treatment of the objects located in the object-holder containers 12 and 13, for example washing, thermal disinfection, sterilization or other means.

The chamber 11 of the treatment machine 10, since it is an example of a "single door" machine, comprises an aperture 17 for inserting the object-holder containers 12, 13 with the objects to be treated and for extracting the object-holder containers 12, 13 with the treated objects.

The aperture 17 will be opened or closed by means of a corresponding door, not shown in the drawings, which can be driven manually or automatically.

The door for opening and closing the aperture 17 can be a sliding door, for example a rolling shutter door or suchlike, and is able to guarantee an adequate seal once the chamber 11 has been closed to carry out the treatment of the objects located in the object-holder containers 12, 13.

Inside the treatment chamber 11, the object-holder containers 12, 13 rest on a pair of supports 18, for example a pair of parallel tracks.

The object-holder containers 12, 13 can also comprise wheels 19 or suchlike in order to move them inside the chamber 11 in direction L.

On at least one bar of the object-holder container, see for example the head bar 15 of the object-holder container 13, a releasable connection device 22 of the object-holder container 13 is positioned with the object-holder container 12 already inserted in the treatment chamber 11.

The connection device 22 substantially comprises a sliding block 23 resting on one side of the bar 15 and provided with at least one attachment element 24, protruding from the other side of the bar 15, that is, the external side of the object-holder container 13.

The attachment element 24 can be, for example, shaped like an upside down U and can have an amplitude such as to be able to sit astride at least two adjacent transverse bars of two object-holder containers 12, 13, for example the bars 15 and 16.

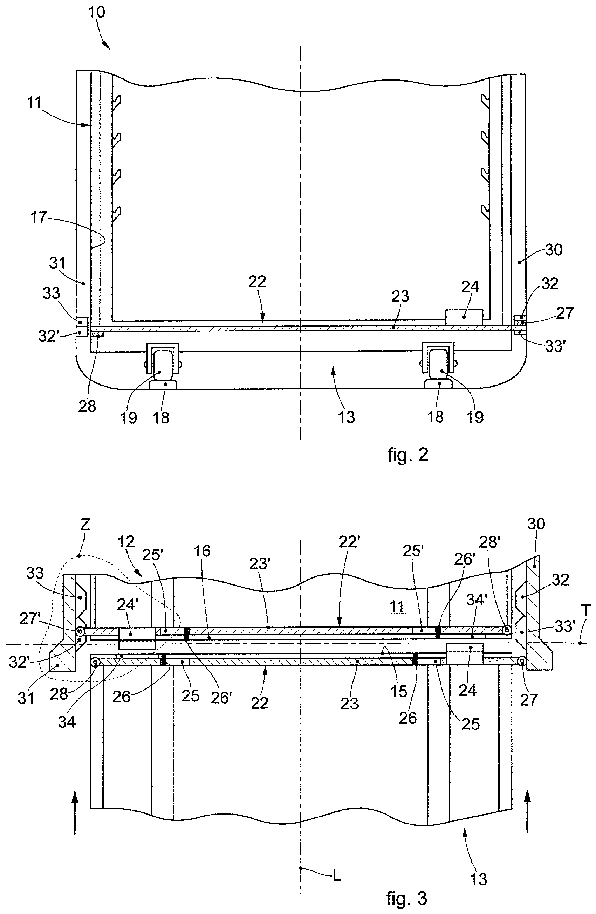

The sliding block 23, see fig. 3, comprises a pair of slits 25, in each of which a pin 26 integral with the bar 15 is inserted. The slits 25 allow the sliding block 23 to slide in direction T, in one sense or the other, with respect to the bar 15. The amplitude of the slits 25 substantially defines the extent of the transverse travel of the sliding block 23.

The sliding block 23 also comprises a rolling element on each end, for example a wheel, see for this purpose the wheels 27 and 28.

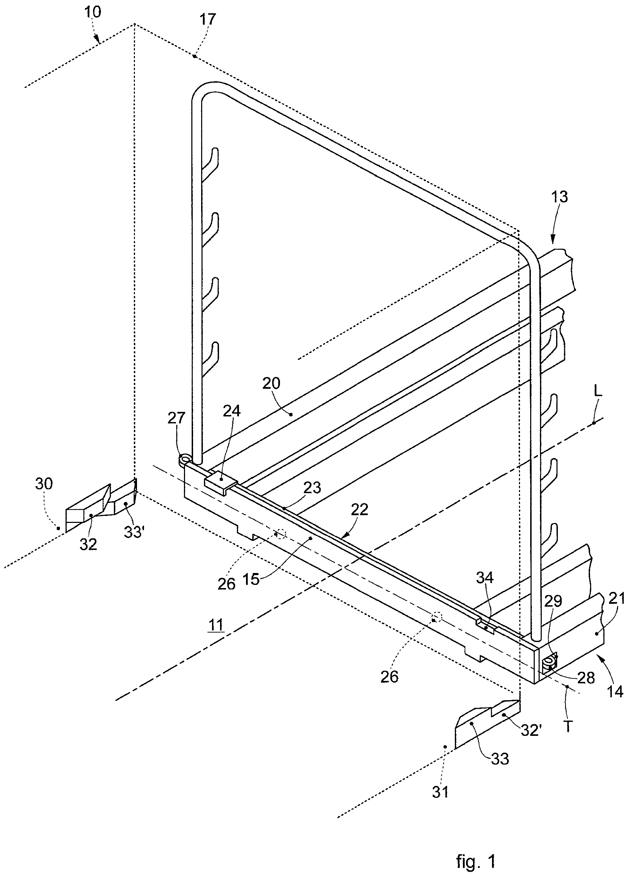

The wheels 27 and 28 are idle and able to protrude alternatively from one side or the other of the object-holder container 13 through suitable holes 29 made in the sides 20 and 21, see for example the hole 29 of fig. 1 made in the side 21 and from which the wheel 28 can exit, as we shall see.

In the example shown, the wheel 27 initially protrudes from the object-holder container 13, while the wheel 28 is housed inside the object-holder container 13.

Preferably, the height at which the wheels 27 and 28 are positioned is different, for example, in the drawings shown, the wheel 27 is situated at a height greater than the wheel 28.

The treatment chamber 11 comprises two opposite lateral walls 30 and 31, on which activation means of the connection device 22 are positioned, for example a cam profile 32 suitably protruding toward the inside of the chamber 11.

The cam profile 32 is positioned at a height such as to cooperate with the wheel 27 protruding from the object-holder container 13, when the object-holder container 13 is inserted in direction L.

Thanks to the cam profile 32, the wheel 27 will be retracted inside the object-holder container 13, determining, as we will see, a transverse activation displacement of the connection device 22 and, in particular, a translation of the sliding block 23 and of the corresponding attachment element 24.

On the lateral wall 31 of the treatment chamber 11 opposite the lateral wall 30, means are positioned to de-activate the connection device 22, for example a cam profile 32', which can be used in the extraction step of the object-holder container. The cam profile 32' also protrudes appropriately toward the inside of the chamber 11.

The cam profile 32' that de-activates the connection device 22, and therefore reciprocally separates the object-holder container 12 and the object-holder container 13, is positioned in proximity to the entrance aperture 17 of the chamber 11, so as to take the object-holder container 12, which is more remote and housed more internally in the chamber 11, toward the exit of the chamber 11, hence in an easily grippable position.

The cam profile 32' is positioned at a height such as to cooperate with the wheel 28, which will protrude from the object-holder container 13 when the object-holder container 13 is removed from the chamber 11.

Thanks to the cam profile 32', the wheel 28 will again be retracted inside the object-holder container 13, determining, as we shall see, a transverse de-activation displacement of the connection device 22 and, in particular, a translation of the sliding block 23 and of the corresponding attachment element 24.

The activation cam profile 32, therefore, in this example, is situated at a greater height than the de-activation cam profile 32', but it could also be the other way round.

In the examples shown, in order to guarantee greater uniformity and symmetry in the connection of the object-holder containers 12 and 13 and in order to provide reciprocal connection devices both at the head and tail of the object-holder containers 12 and 13, another connection device 22' is positioned in the tail bar 16 of the object-holder container already inserted in the treatment chamber 11.

The provision of connection devices 22 and 22' at both the head and tail of the object-holder containers 12 and 13 also allows the operator not to confuse the head part with the tail part of the object-holder container 12 or 13, since the head and tail parts will be reciprocally symmetrical.

Structurally, the connection device 22' is very similar to the connection device 22 associated with the bar 15 of the object-holder container 13.

When the connection of the object-holder containers 12 and 13 is complete, preferably, the attachment elements 24 and 24' of the connection devices 22 and 22' will be disposed in specular positions with respect to the longitudinal axis L.

The connection device 22' will therefore be provided with a sliding block 23', at the ends of which corresponding wheels 27' and 28' are positioned.

Since the wheel 27 of the connection device 22 associated with the object-holder container 13 initially protrudes from the object-holder container 13, the wheel 28' of the object-holder container 12, which is on the same side of the wheel 27, will be housed inside the object-holder container 12.

In the same way, since the wheel 27' of the connection device 22' associated with the object-holder container 12 initially protrudes from the object-holder container 12, the wheel 28 of the object-holder container 13, which is on the same side of the wheel 27', will initially be inserted into the object-holder container 13.

The wheel 27' of the connection device 22', which in this example is situated at a greater height than the wheel 28', is therefore able to cooperate with a cam profile 33 to activate the connection device 22'. The cam profile 33 suitably protrudes toward the inside of the chamber 11.

The cam profile 33 is positioned on the lateral wall 31 of the treatment chamber 11 in a longitudinally staggered position with respect to the position of the cam profile 32', so as to allow the alternate drive of the wheels 27' of the object-holder container 12 and of the wheel 28 of the object-holder container 13.

The wheel 28' of the connection device 22' is instead able to cooperate with a cam profile 33' to de-activate the connection device 22'. The cam profile 33' suitably protrudes toward the inside of the chamber 11.

Like the de-activation cam profile 32', the cam profile 33' to de-activate the connection device 22' and thus to separate the object-holder containers 12 and 13 is disposed in proximity to the entrance or exit aperture 17 of the treatment chamber 11.

The cam profile 33' is positioned on the lateral wall 30 of the treatment chamber 11 in a longitudinally staggered position with respect to the position of the cam profile 32, so as to allow the alternate drive of the wheels 28' of the object-holder container 12 and of the wheel 27 of the object-holder container 13.

In the same way as seen for the connection device 22, also the sliding block 23' of the connection device 22' is provided with slits 25' able to be engaged by pins 26' integral with the bar 16, in this case the tail bar of the object-holder container 12.

Substantially therefore, the activation means of the connection devices 22 and 22', comprising for example the cam profiles 32 and 33, are positioned at the same height and in the same longitudinal position in the chamber 11. Similarly, the de-activation means of the connection devices 22 and 22', for example comprising the cam profiles 32' and 33', are also positioned at the same height and in the same longitudinal position in the chamber 11.

The activation cam profiles 32 and 33, as we have seen, are longitudinally staggered with respect to the cam profiles 32' and 33'.

The cam profiles 32 and 33 are also situated at a greater height than the cam profiles 32' and 33'.

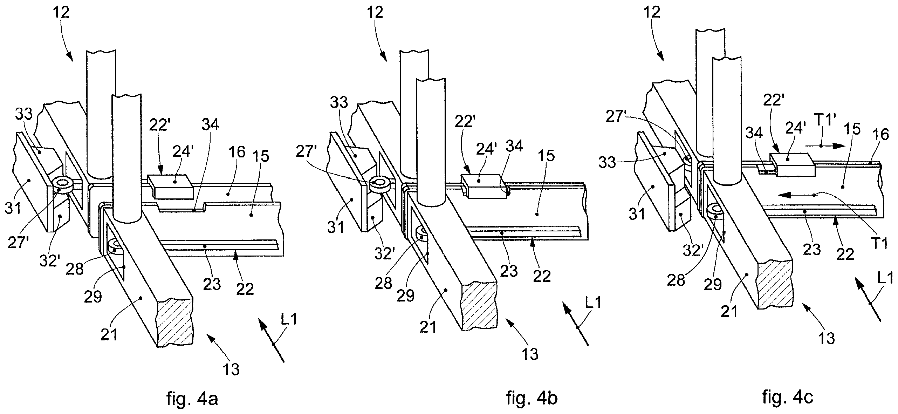

The sequence of connection and disconnection of the object-holder containers 12 and 13 shown schematically in figs. 4a-4d substantially relates to the zone Z circled in dashed lines in fig. 3.

Let us suppose that the object-holder container 12 is already inserted in the treatment chamber 11.

The object-holder container 13, for example by means of a rollerway or suchlike, is moved into proximity to the entrance aperture 17 of the treatment chamber 11, as can be seen for example also in figs. 1 or 3.

The rollerway or other approach and removal unit outside the treatment chamber 11 can be provided with movement means with respect to the chamber 11, for example translation means, so as to be able to discharge the object-holder containers 12 and 13 extracted from the chamber 11.

The head bar 15 of the object-holder container 13 is provided with at least a groove 34 of an amplitude such as to allow the temporary passage of the attachment element 24' of the connection device 22' associated with the object-holder container 12.

Similarly, the tail bar 16 of the object-holder container 12 is also provided with a groove 34' of an amplitude such as to allow the temporary passage of the attachment element 24 of the connection device 22 associated with the object-holder container 13.

The object-holder container 13 is thrust in direction L1 inside the treatment chamber 11 until the bar 15 and the bar 16 are reciprocally adjacent, see fig. 4b. As can be seen, the attachment element 24' is positioned above both the bars 15 and 16 and has crossed the groove 34 of the bar 15. Similarly, the attachment element 24 will have passed the groove 34' of the bar 16 and will find itself above both bars 15 and 16.

Continuing in the thrust of the object-holder container 13 inside the treatment chamber 11, the object-holder container 12 will move toward the bottom of the chamber 11.

When the protruding wheel 27' of the connection device 22' meets the cam profile 33, fig. 4c, the connection device 22' and the corresponding attachment element 24' are automatically translated in direction T1', that is, in this case, toward the center of the chamber 11. Similarly, when the wheel 27' cooperates with the cam profile 33, the wheel 27 of the connection device 22 cooperates with the cam profile 32, so that the device 22 moves automatically in a direction T1, opposite to the direction T1', thus also toward the center of the chamber 11. In this situation, the wheel 28 of the connection device 22 begins to exit from the hole 29 and to protrude out of the object-holder container 13.

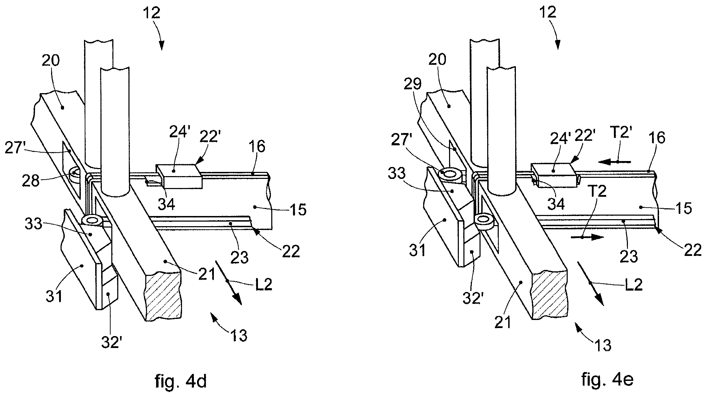

The translation T1 and T1' of the two connection devices 22 and 22' causes the automatic overlapping of the attachment elements 24 and 24' on both the bars 15 and 16, as shown for example in fig. 4d for the attachment element 24'.

When the positioning is completed, as previously mentioned, the attachment elements 24 and 24' will be substantially in a symmetrical position with respect to the longitudinal axis L.

At this point, therefore, the object-holder containers 12 and 13 are attached to each other and the first object-holder container 12 can be taken, always thrusting the object-holder container 13, to the bottom of the treatment chamber 11.

In the extraction step, the object-holder container inserted last, hence the object-holder container 13, is translated in the direction L2, see fig. 4d again, and draws with it the first object-holder container inserted in the chamber 11, that is, the object-holder container 12, since they are reciprocally connected.

Continuing the extraction translation L2, the protruding wheel 28 of the connection device 22 does not interact with the activation cam profile 33, in this case it passes under the cam profile 33 and instead interacts with the de-activation cam profile 32, thus determining an automatic translation in direction T2, see fig. 4e, of the connection device 22.

Similarly, the wheel 28' of the connection device 22' will cooperate with the cam profile 33' so as to automatically retract and translate the connection device 22' in direction T2', and then return the wheel 27' in a position protruding from the object-holder container 12.

The translation T2' will preferably be the same entity and opposite to the translation T1', and the translation T2 will preferably be the same entity and opposite to the translation T1.

The translations T2 and T2' substantially determine the release of the attachment elements 24 and 24' and the return of the object-holder containers 12 and 13 to the situation of fig. 4b, so in this situation the object-holder containers 12 and 13 are again released. The attachment elements 24 and 24' are in fact again in correspondence with the grooves 34 and 34' made on the bars 15 and 16. Continuing in the extraction in direction L2, one passes from the situation of fig. 4b to the situation of fig. 4a.

As mentioned initially, it would also be possible to provide only one connection device 22 or 22', for example imagining that the attachment element 24 or 24' is automatically activated at entrance in order to sit astride both the bars 15 and 16 of the object-holder containers 12 and 13, determining the connection thereof and possibly de-activating automatically at exit, allowing the separation thereof. The automatic activation of the connection device 22 or 22' will be determined, for example, by the cam profile 32 or 33 and its possible automatic de-activation will be determined by the cam profile 32' or 33'.

It is important that the connection device 22 and/or 22' is provided with means which allow its automatic activation, in order to connect the object-holder containers 12 and 13 at the moment of insertion into the chamber 11 and to be able to extract them together once inside the chamber 11. The de-activation of the reciprocal connection of the object-holder containers 12 and 13 can preferably take place automatically and in correspondence with the entrance or exit aperture 17 of the treatment chamber 11, so that the first of the object-holder containers inserted, that is, the object-holder container 12 located furthest down the chamber 11, is, at the moment of separation from the second object-holder container 13, in proximity to the entrance aperture 17 of the chamber 11. This separation could however also take place manually, after the extraction of the object-holder containers 12 and 13.

The activation means of the connection devices 22 and/or 22', and hence the reciprocal connection of the object-holder containers 12 and 13, for example the cam profiles 32 and 33, could also be disposed in more internal positions along the lateral walls 30 and 31 of the treatment chamber 11, provided, substantially, they are within a distance smaller than the travel of the object-holder containers 12 and 13 in the treatment chamber 11.

In fact, in the insertion step, the object-holder containers 12 and 13 do not need to be reciprocally joined, the reciprocal connection between them is necessary in the extraction step, when pulling the outermost object-holder container it must also be possible to pull out the innermost container.

The present treatment machine 10 is therefore equipped with one or more connection devices 22 and 22' and with activation and possible de-activation means, that is, for example the cam profiles 32, 32', 33, 33', of an essentially mechanical type, therefore able to operate even in onerous and difficult operating conditions, such as those of a sterilization machine where the operating temperatures can also be quite high, even up to 140°C and where, therefore, it is inadvisable to use motorized and/or electric or electronic means for the reciprocal and automatic connection of the object-holder containers inside the chamber.

As previously mentioned, moreover, the object-holder containers 12 and 13 can be disposed outside the chamber on a rollerway or other guide and support system to be inserted into and removed from the chamber 11. As we said, the rollerway or other guide system could be mobile with respect to the treatment chamber 11, so as to be able to be displaced for example, in order to move the object-holder containers 12 and 13 located on the rollerway to a zone different from that in which the treatment chamber 11 is located.

In the following claims, the sole purpose of the references in brackets is to facilitate reading: they must not be considered as restrictive factors with regard to the field of protection claimed in the specific claims.

## Claims

1. Machine for treating objects, in particular washing, thermal disinfection and/or sterilization of objects, comprising at least a treatment chamber (11) configured to house a plurality of object-holder containers (12, 13) located in sequence and comprising opposite lateral walls (30, 31), **characterized in that** it comprises:
at least a releasable connection device (22, 22') of said object-holder containers (12, 13), associated with at least one of the object-holder containers (12, 13);
activations means (32, 33) positioned on said lateral walls (30, 31) inside the treatment chamber (11) and configured to cooperate with said releasable connection device (22, 22');;
said device (22, 22') comprising at least a sliding block (23, 23') associated with at least a first head and/or tail bar (15, 16) of an object-holder container (13, 12) and provided with an attachment element (24, 24') able to engage said first bar (15, 16) and at least a second bar (16, 15) of another object-holder container (12, 13), following the translation of said sliding block (23, 23') with respect to said first bar (15, 16), said sliding block (23, 23') being made to translate in one direction or the other with respect to said bar (15, 16) by means of said activation means (32, 33) so that, when inserting an object-holder container (13) in the treatment chamber (11) after at least a first object-holder container (12) already inserted in said chamber (11) and moving said object-holder containers (12, 13) toward the inside of the chamber (11), said releasable connection device (22, 22') is automatically activated by said activation means (32, 33) in order to reciprocally connect said object-holder containers (12, 13), so as to allow a simultaneous extraction, acting on the last object-holder container (13) inserted;
and further comprising de-activation means (32', 33') of said releasable connection device (22, 22'), so as to automatically separate said object-holder containers (12, 13) during an extraction step of the object-holder containers (12, 13) from the chamber (11);
wherein said sliding block (23, 23') comprises at one end at least a first rolling element (27, 27') able to engage with said activation means (32, 33) and, at the other end, at least a second rolling element (28, 28') able to engage with said de-activation means (32', 33');
wherein said first and second rolling elements (27, 27', 28, 28') are alternatively protruding from opposite sides (20, 21) of the object-holder container (12, 13); and
wherein said de-activation means comprise at least a cam profile (32', 33') which can engage with said second rolling element (28, 28') of said releasable connection device (22, 22').

2. Machine as in claim 1, **characterized in that** said de-activation means (32', 33') are positioned in proximity to an entrance aperture (17) of the treatment chamber (11).

3. Machine as in any claim hereinbefore, **characterized in that** said releasable connection device (22, 22') is provided both at the tail and at the head of each object-holder container (12, 13).

4. Machine as in claim 3, **characterized in that** the activation means (32, 33) and the de-activation means (32', 33') of said connection devices (22, 22') are staggered at least in height in said lateral walls (30, 31) inside said treatment chamber (11).

5. Machine as in claim 1, **characterized in that** said activation means comprise at least a cam profile (32, 33) which can engage with said first rolling element (27, 27') of said releasable connection device (22, 22').

6. Method to connect a plurality of object-holder containers located in sequence in a treatment machine for washing, thermal disinfection and/or sterilization of objects as in any claim hereinbefore, **characterized in that** it provides: to insert a first object-holder container (12) in a treatment chamber (11); to insert at least a second object-holder container (13) in the treatment chamber (11) after said first object-holder container (12) which has already been inserted in said treatment chamber (11), and to move said object-holder containers (12, 13) toward the inside of the treatment chamber (11); at least one of said object-holder containers (12, 13) being provided with a releasable connection device (22, 22') automatically activated by activation means (32, 33) disposed on said lateral walls (30, 31) inside the treatment chamber (11) to reciprocally connect said object-holder containers (12, 13), so as to allow a simultaneous extraction, acting on the last object-holder container (13) inserted, said device (22, 22') comprising at least a sliding block (23, 23') associated with at least a first head and/or tail bar (15, 16) of an object-holder container (13, 12) and provided with an attachment element (24, 24') able to engage said first bar (15, 16) and at least a second bar (16, 15) of another object-holder container (12, 13), following a translation of said sliding block (23, 23') with respect to said first bar (15, 16), said sliding block (23, 23') being made to translate in one direction or the other with respect to said bar (15, 16) by means of activation means (32, 33).

## Patentansprüche

1. Maschine zur Behandlung von Objekten, insbesondere zur Reinigung, thermischen Desinfektion und/oder Sterilisation von Objekten, die mindestens eine Behandlungskammer (11) umfasst, die so ausgebildet ist, dass sie eine Vielzahl von Objekthaltecontainern (12, 13) aufnimmt, die nacheinander angeordnet sind und gegenüberliegende Seitenwände (30, 31) aufweisen, **dadurch gekennzeichnet, dass** sie Folgendes umfasst:
mindestens eine lösbare Verbindungseinrichtung (22, 22') der Objekthaltecontainer (12, 13), die mit mindestens einem der Objekthaltecontainer (12, 13) verbunden ist;
Aktivierungsmittel (32, 33), die an den Seitenwänden (30, 31) im Inneren der Behandlungskammer (11) angeordnet und so ausgebildet sind, dass sie mit der lösbaren Verbindungseinrichtung (22, 22') zusammenwirken;
wobei die Vorrichtung (22, 22') mindestens einen Gleitblock (23, 23') umfasst, der mit mindestens einer ersten Kopf- und/oder Heckstange (15, 16) eines Objekthaltecontainers (13, 12) verbunden und mit einem Befestigungselement (24, 24') ausgestattet ist, das in der Lage ist, mit der ersten Stange (15, 16) und mindestens einer zweiten Stange (16, 15) eines weiteren Objekthaltecontainers (12, 13) nach der Verschiebung des Gleitblocks (23, 23') in Bezug auf die erste Stange (15, 16) in Eingriff zu kommen, wobei der Gleitblock (23, 23') dazu ausgebildet ist, sich in der einen oder der anderen Richtung in Bezug auf die Stange (15, 16) mittels der Aktivierungsmittel (32, 33) zu verschieben, so dass beim Einsetzen eines Objekthaltecontainers (13) in die Behandlungskammer (11) nach mindestens einem ersten, bereits in die Kammer (11) eingesetzten Objekthaltecontainer (12) und beim Bewegen der Objekthaltecontainer (12, 13) in das Innere der Kammer (11) die lösbare Verbindungseinrichtung (22, 22') durch die Aktivierungsmittel (32, 33) automatisch aktiviert wird, um die Objekthaltecontainer (12, 13) gegenseitig zu verbinden, sodass eine gleichzeitige Entnahme möglich ist, die an dem zuletzt eingesetzten Objekthaltecontainer (13) durchgeführt wird;
und ferner Deaktivierungsmittel (32', 33') der lösbaren Verbindungseinrichtung (22, 22') umfasst, um die Objekthaltecontainer (12, 13) während eines Entnahmeschrittes der Objekthaltecontainer (12, 13) von der Kammer (11) automatisch zu trennen;
wobei der Gleitblock (23, 23') an einem Ende mindestens ein erstes Rollenelement (27, 27') aufweist, das in der Lage ist, mit den Aktivierungsmitteln (32, 33) in Eingriff zu kommen, und an dem anderen Ende mindestens ein zweites Rollenelement (28, 28') aufweist, das in der Lage ist, mit den Deaktivierungsmitteln (32', 33') in Eingriff zu kommen;
wobei das erste und das zweite Rollenelement (27, 27', 28, 28') von gegenüberliegenden Seiten (20, 21) des Objekthaltecontainers (12, 13) abwechselnd vorstehen; und
wobei die Deaktivierungsmittel mindestens ein Nockenprofil (32', 33') umfassen, das mit dem zweiten Rollenelement (28, 28') der auslösbaren Verbindungseinrichtung (22, 22') in Eingriff kommen kann.

2. Maschine nach Anspruch 1, **dadurch gekennzeichnet, dass** die Deaktivierungsmittel (32', 33') in der Nähe einer Eintrittsöffnung (17) der Behandlungskammer (11) angeordnet sind.

3. Maschine nach einem der vorstehenden Ansprüche, **dadurch gekennzeichnet, dass** die lösbare Verbindungseinrichtung (22, 22') sowohl am Heck als auch am Kopf jedes Objekthaltecontainers (12, 13) vorgesehen ist.

4. Maschine nach Anspruch 3, **dadurch gekennzeichnet, dass** die Aktivierungsmittel (32, 33) und die Deaktivierungsmittel (32', 33') der Verbindungseinrichtungen (22, 22') in den Seitenwänden (30, 31) innerhalb der Behandlungskammer (11) zumindest in der Höhe versetzt angeordnet sind.

5. Maschine nach Anspruch 1, **dadurch gekennzeichnet, dass** die Aktivierungsmittel mindestens ein Nockenprofil (32, 33) umfassen, das in das erste Rollenelement (27, 27') der lösbaren Verbindungseinrichtung (22, 22') eingreifen kann.

6. Verfahren zum Kombinieren einer Vielzahl von Objekthaltecontainern, die nacheinander in einer Behandlungsmaschine zur Reinigung, thermischen Desinfizierung und/oder Sterilisierung von Objekten angeordnet sind, nach jedem der vorstehenden Ansprüche, **dadurch gekennzeichnet, dass** es Folgendes zur Verfügung stellt: Einsetzen eines ersten Objekthaltecontainers (12) in eine Behandlungskammer (11); Einsetzen mindestens eines zweiten Objekthaltecontainers (13) in die Behandlungskammer (11) nach dem ersten Objekthaltecontainer (12), der bereits in die Behandlungskammer (11) eingesetzt wurde, und Bewegen der Objekthaltecontainer (12, 13) in das Innere der Behandlungskammer (11); wobei mindestens einer der Objekthaltecontainer (12, 13) mit einer lösbaren Verbindungseinrichtung (22, 22') ausgestattet ist, die durch Aktivierungsmittel (32, 33) automatisch aktiviert wird, die an den Seitenwänden (30, 31) innerhalb der Behandlungskammer (11) angeordnet sind, um die Objekthaltecontainer (12, 13) gegenseitig zu verbinden, sodass eine gleichzeitige Entnahme zu ermöglichen wird, die an dem zuletzt eingesetzten Objekthaltecontainer (13) durchgeführt wird, wobei die Vorrichtung (22, 22') mindestens einen Gleitblock (23, 23') umfasst, der mit mindestens einer ersten Kopf- und/oder Heckstange (15, 16) eines Objekthaltecontainers (13, 12) verbunden und mit einem Befestigungselement (24, 24') ausgestattet ist, das in der Lage ist, mit der ersten Stange (15, 16) und mindestens einer zweiten Stange (16, 15) eines weiteren Objekthaltecontainers (12, 13) nach einer Verschiebung des Gleitblocks (23, 23') in Bezug auf die erste Stange (15, 16) in Eingriff zu kommen, wobei der Gleitblock (23, 23') dazu ausgebildet ist, sich in der einen oder der anderen Richtung in Bezug auf die Stange (15, 16) mittels der Aktivierungsmittel (32, 33) zu verschieben.

## Revendications

1. Machine de traitement d'objets, en particulier de lavage, de désinfection thermique et/ou de stérilisation d'objets, comprenant au moins une chambre de traitement (11) configurée pour loger une pluralité de contenants de support d'objets (12, 13) situés en séquence et comportant des parois latérales opposées (30, 31), **caractérisée en ce qu'**elle comprend :
au moins un dispositif de connexion libérable (22, 22') desdits contenants de support d'objets (12, 13), associé à au moins un des contenants de support d'objets (12, 13) ;
des moyens d'activation (32, 33) positionnés sur lesdites parois latérales (30, 31) à l'intérieur de la chambre de traitement (11) et configurés pour coopérer avec ledit dispositif de connexion libérable (22, 22') ;
ledit dispositif (22, 22') comprenant au moins un bloc coulissant (23, 23') associé à au moins une première barre de tête et/ou de queue (15, 16) d'un contenant de support d'objets (13, 12) et prévu avec un élément de fixation (24, 24') apte à coopérer avec ladite première barre (15, 16) et au moins une seconde barre (16, 15) d'un autre contenant de support d'objets (12, 13), suite au déplacement en translation dudit bloc coulissant (23, 23') par rapport à ladite première barre (15, 16), ledit bloc coulissant (23, 23') étant amené à se déplacer en translation dans une direction ou dans l'autre par rapport à ladite barre (15, 16) par l'intermédiaire desdits moyens d'activation (32, 33) de sorte que, lors de l'insertion d'un contenant de support d'objets (13) dans la chambre de traitement (11) après au moins un premier contenant de support d'objets (12) déjà inséré dans ladite chambre (11) et en déplaçant lesdits contenants de support d'objets (12, 13) vers l'intérieur de la chambre (11), ledit dispositif de connexion libérable (22, 22') est activé automatiquement par lesdits moyens d'activation (32, 33) afin de connecter réciproquement lesdits contenants de support d'objets (12, 13), de manière à permettre une extraction simultanée, en agissant sur le dernier contenant de support d'objets (13) inséré ;
et comprenant en outre des moyens de désactivation (32', 33') dudit dispositif de connexion libérable (22, 22'), de manière à séparer automatiquement lesdits contenants de support d'objets (12, 13) lors d'une étape d'extraction des contenants de support d'objets (12, 13) à partir de la chambre (11) ;
dans lequel ledit bloc coulissant (23, 23') comprend à une première extrémité au moins un premier élément roulant (27, 27') apte à coopérer avec lesdits moyens d'activation (32, 33) et, à l'autre extrémité, au moins un second élément roulant (28, 28') apte à coopérer avec lesdits moyens de désactivation (32', 33') ;
dans lequel lesdits premier et second éléments roulants (27, 27', 28, 28') font en variante saillie depuis des côtés opposés (20, 21) du contenant de support d'objet (12, 13) ; et
dans lequel lesdits moyens de désactivation comprennent au moins un profil de came (32', 33') qui peut coopérer avec ledit second élément roulant (28, 28') dudit dispositif de connexion libérable (22, 22').

2. Machine selon la revendication 1, **caractérisée en ce que** lesdits moyens de désactivation (32', 33') sont positionnés à proximité d'une ouverture d'entrée (17) de la chambre de traitement (11).

3. Machine selon l'une quelconque des revendications précédentes, **caractérisée en ce que** ledit dispositif de connexion libérable (22, 22') est prévu à la fois en queue et en tête de chaque contenant de support d'objets (12, 13).

4. Machine selon la revendication 3, **caractérisée en ce que** les moyens d'activation (32, 33) et les moyens de désactivation (32', 33') desdits dispositifs de connexion (22, 22') sont étagés au moins en hauteur dans lesdites parois latérales (30, 31) à l'intérieur de ladite chambre de traitement (11).

5. Machine selon la revendication 1, **caractérisée en ce que** lesdits moyens d'activation comprennent au moins un profil de came (32, 33) qui peut coopérer avec ledit premier élément roulant (27, 27') dudit dispositif de connexion libérable (22, 22').

6. Procédé pour connecter une pluralité de contenants de support d'objets disposés en séquence dans une machine de traitement pour le lavage, la désinfection thermique et/ou la stérilisation d'objets selon l'une quelconque des revendications précédentes, **caractérisé en ce qu'**il comporte les étapes consistant à : insérer un premier contenant de support d'objets (12) dans une chambre de traitement (11) ; insérer au moins un second contenant de support d'objets (13) dans la chambre de traitement (11) après ledit premier contenant de support d'objets (12) qui a déjà été inséré dans ladite chambre de traitement (11), et déplacer ledit contenant de support d'objets (12, 13) vers l'intérieur de la chambre de traitement (11) ; au moins un desdits contenants de support d'objets (12, 13) étant pourvu d'un dispositif de connexion libérable (22, 22') activé automatiquement par des moyens d'activation (32, 33) disposés sur lesdites parois latérales (30, 31) à l'intérieur de la chambre de traitement (11) pour connecter réciproquement lesdits contenants de support d'objets (12, 13), de manière à permettre une extraction simultanée, en agissant sur le dernier contenant de support d'objets (13) inséré, ledit dispositif (22, 22') comprenant au moins un bloc coulissant (23, 23') associé à au moins une première barre de tête et/ou de queue (15, 16) d'un contenant de support d'objets (13, 12) et muni d'un élément de fixation (24, 24') apte à coopérer avec ladite première barre (15, 16) et au moins une seconde barre (16, 15) d'un autre contenant de support d'objets (12, 13), suite à un déplacement en translation dudit bloc coulissant (23, 23') par rapport à ladite première barre (15, 16), ledit bloc coulissant (23, 23') étant amené à se déplacer en translation dans une direction ou dans l'autre par rapport à ladite barre (15, 16) par l'intermédiaire de moyens d'activation (32, 33).
